# EUROPEAN PATENT APPLICATION

(11) **EP 4 796 138 A1**
(43) Date of publication of application: **26.08.2026**
(21) Application number: 24878384.7
(22) Date of filing: 09.04.2024
(51) Int. Cl.: A61M 1/00, A61B 90/00, A61G 12/00, A61B 50/13, B08B 9/093

(54) **WASTE COLLECTION DEVICE, WASTE LIQUID COLLECTION AND TREATMENT SYSTEM, AND DOCKING METHOD FOR WASTE LIQUID COLLECTION AND TREATMENT SYSTEM**

(30) Priority: 16.10.2023 CN 202311345997; 16.10.2023 CN 202322776172 U
(71) Applicant: Amsino Medical (Shanghai) Co., Ltd., Shanghai 201613 (CN)
(72) Inventor: LU, Lei, Shanghai 201613 (CN); ZENG, Qingling, Shanghai 201613 (CN); LI, Ya, Shanghai 201613 (CN)
(74) Representative: Metida
(86) International application number: PCT/CN2024/086895
(87) International publication number: WO 2025/081730

(57) **Abstract**

Disclosed in this invention are a waste collection device, a waste liquid collection and treatment system, and a docking method for the waste liquid collection and treatment system. The waste collection device is used for docking with a docking device (30) and comprises a plurality of collection joints; the plurality of collection joints comprise a liquid discharge female joint (1601), a flushing female joint (1602) and a steam female joint (1603); the liquid discharge female joint (1601) and the flushing female joint (1602) are arranged in a floating mode; the docking device (30) comprises a plurality of docking joints; the plurality of docking joints comprise a liquid discharge male joint (3401), a flushing male joint (3402) and a steam male joint (3403). According to the waste collection device, the waste liquid collection and treatment system, and the docking method for the waste liquid collection and treatment system, steam cleaning can be carried out on the waste liquid collection and treatment system, avoiding the use of a large amount of hot water, and this invention can be widely used in various medical units having different conditions; when coupling between the liquid discharge joints and coupling between the flushing joints are completed, coupling between the steam female joint and the steam male joint is started, so that the liquid discharge joints and the flushing joints can achieve a positioning effect on coupling between the steam joints, avoiding the problem of low fault tolerance when the three sets of joints are coupled at the same time, and also improving the coupling success rate.

## Description

This application claims the priority of Chinese patent application No. 202311345997.X, entitled "waste collection device, waste liquid collection and treatment system, and docking method therefor", and Chinese patent application No. 202322776172.5, entitled "waste collection device and waste liquid collection and treatment system", both filed on October 16, 2023, the entire contents of which are incorporated herein by reference.

### TECHNICAL FIELD

This invention relates to the technical field of medical devices, and in particular to a waste collection device, a waste liquid collection and treatment system, and a docking method for a waste liquid collection and treatment system.

### BACKGROUND

During certain surgical procedures, various types of waste such as liquid, semi-solid and solid waste are inevitably generated, for example, human fluids such as blood, irrigation solutions introduced into the surgical site during surgery, human tissue fragments, small pieces of surgical materials, and other solid and semi-solid waste produced during surgery. Such waste needs to be collected promptly to avoid contaminating the surgical site and to prevent it from becoming a biological hazard in the operating room or other locations where surgery is performed. In the prior art, suction force is usually generated by a vacuum source to aspirate waste generated at the surgical site into a specific collection container. That is, after the system is activated, the suction force generated by the vacuum source reaches the surgical site, thereby drawing waste into the specific collection container through pipelines connected to the surgical site.

In one medical waste collection and treatment system, waste materials are collected in a waste collection container connected to a vacuum source. The waste collection container is generally mounted on a portable trolley with wheels for easy movement and transportation. Generally, during the suction process, the waste collection container needs to be emptied when its storage volume reaches a predetermined volume. In the early prior art, the waste collection device is pushed to a docking station to be emptied and cleaned. The waste collection unit starts emptying after docking with the docking station, and once emptied, the waste collection container is cleaned by a cleaning system through disinfection and washing.

### SUMMARY

### Technical Problem

In a series of medical procedures, emptying is required for each procedure, and the frequent operation of pushing the waste collection device to the docking station each time causes much inconvenience to users and affects medical progress. Therefore, to address this technical drawback, transfer devices specially used for transporting waste from the waste collection device to the docking station have been developed, namely docking devices dedicated to transporting waste in the waste collection device. Such devices generally include two connectors docked with two connectors of the waste collection device to form fluid passages, one fluid passage for transferring waste from the waste collection device to the docking device, and the other fluid passage for cleaning the waste collection container of the waste collection device. However, cleaning usually requires hot water above 40°C, and many hospitals cannot provide such a large amount of hot water.

### Technical Solution

To overcome the drawbacks and deficiencies existing in the prior art, an object of this invention is to provide a waste collection device, a waste liquid collection and treatment system, and a docking method for a waste liquid collection and treatment system, which can realize high-temperature cleaning without requiring a large amount of hot water.

The object of this invention is achieved through the following technical solutions: this invention provides a waste collection device for docking with a docking device, wherein the waste collection device includes a trolley docking portion, the trolley docking portion includes a trolley connector mounting plate and a plurality of collection connectors arranged on the trolley connector mounting plate, the plurality of collection connectors include a liquid discharge female connector, a flushing female connector and a steam female connector, and the liquid discharge female connector and the flushing female connector are arranged to be floating relative to the trolley connector mounting plate.

In one embodiment, the steam female connector is arranged to be floating relative to the trolley connector mounting plate.

In one embodiment, there is one liquid discharge female connector and one flushing female connector respectively, the liquid discharge female connector and the flushing female connector are arranged side by side in a direction perpendicular to the relative movement direction when the docking device is docked with the waste collection device, and there are two steam female connectors arranged side by side in a direction perpendicular to the relative movement direction when the docking device is docked with the waste collection device.

In one embodiment, the steam female connector is closer to an outer side of the waste collection device than the liquid discharge female connector and the flushing female connector.

In one embodiment, the ends of the liquid discharge female connector and the flushing female connector for docking with the docking device are flush, and the end of the steam female connector for docking with the docking device is higher than the ends of the liquid discharge female connector and the flushing female connector for docking with the docking device.

In one embodiment, each of the liquid discharge female connector and the flushing female connector includes a collection-end connector and a first floating connector, the first floating connector is sleeved on the collection-end connector and mounted on the trolley connector mounting plate, and the first floating connector is movable in a floating manner relative to the trolley connector mounting plate; the steam female connector includes a collection-end mounting base and a second floating connector, the collection-end mounting base is sleeved inside the second floating connector, the second floating connector is mounted on the trolley connector mounting plate, and the second floating connector is movable in a floating manner relative to the trolley connector mounting plate.

In one embodiment, the trolley docking portion is provided with a mating space for accommodating the mating head of the docking device; the collection-end connector and the first floating connector are fixedly connected to form a whole, the trolley docking portion further includes a first floating connector mounting member, the first floating connector includes a first floating port and a first floating guide member, the first floating port is fixedly connected to the first floating guide member, the first floating connector mounting member is fixed to the trolley connector mounting plate, the first floating guide member is sleeved on the collection-end connector and penetrates the first floating connector mounting member, the first floating port is floatingly matched with a mounting hole of the first floating connector mounting member, and a first elastic member is arranged between the first floating connector mounting member and the first floating connector; the trolley docking portion further includes a second floating connector mounting member, the second floating connector includes a second floating port and a second floating guide member, the collection-end mounting base and the second floating connector are fixedly connected to form a whole, the second floating connector mounting member is fixed to the trolley connector mounting plate, the second floating guide member is sleeved on the collection-end mounting base and penetrates the second floating connector mounting member, the second floating port is fixedly connected to the second floating guide member, and a second elastic member is arranged between the second floating connector mounting member and the second floating connector.

In one embodiment, a first matching hole is formed on the first floating connector mounting member, the first floating port includes a matching surface for matching with the first matching hole of the first floating connector mounting member, and the first matching hole is a tapered hole with a large upper part and a small lower part, or an inner side wall of the first matching hole is an inclined plane; a second matching hole is formed on the second floating connector mounting member, the second floating port includes a matching surface for matching with the second matching hole of the second floating connector mounting member, and the second matching hole is a tapered hole with a large upper part and a small lower part, or an inner side wall of the second matching hole is an inclined plane.

In one embodiment, the first floating guide member includes a first sleeve portion and a first guide end, one end of the first sleeve portion is connected to the collection-end connector, the first guide end is connected to the other end of the first sleeve portion, a first guide opening is formed in the first guide end, and the first guide opening is a bell-mouth structure; the second floating guide member includes a second sleeve portion, a second guide end and a connecting portion, the second guide end and the connecting portion are respectively connected to two ends of the second sleeve portion, the collection-end mounting base is connected inside the second sleeve portion, a second guide opening is formed in the second guide end, and the second guide opening is a bell-mouth structure.

In one embodiment, the first floating connector mounting member includes a positioning portion extending into the mating space, the first floating guide member further includes a first limiting edge cooperating with the positioning portion to limit an upward floating range of the first floating connector during floating, and the first limiting edge is arranged on an outer side wall of the first guide end; or, the first floating guide member further includes an outer tube, the outer tube is connected to the outer side wall of the first guide end, and the outer tube is sleeved outside the first guide end with a gap formed therebetween.

This invention further provides a waste collection device, including the above waste collection device and a docking device for docking with the waste collection device, wherein the docking device includes a mating head, the mating head includes a plurality of docking connectors, and the plurality of docking connectors include a liquid discharge male connector, a flushing male connector and a steam male connector arranged corresponding to the liquid discharge female connector, the flushing female connector and the steam female connector respectively.

In one embodiment, the mating head further includes a mating plate, two guide slots for the liquid discharge female connector and the flushing female connector to enter are formed in the mating plate, the guide slot is U-shaped, an inner end of the guide slot is arc-shaped, and side edges of the guide slot are wedge-shaped.

In one embodiment, the mating head further includes a moving plate and an actuating assembly, the liquid discharge male connector, the flushing male connector and the steam male connector are all arranged on the moving plate, and the actuating assembly is connected to the moving plate and configured to drive the moving plate to move up and down.

This invention further provides a docking method for a waste liquid collection and treatment system, for docking the waste collection device and the docking device of the above waste liquid collection and treatment system. The docking method for a waste liquid collection and treatment system includes:
moving the waste collection device and the docking device toward each other;
aligning the mating head with the trolley docking portion, such that the liquid discharge female connector, the flushing female connector and the steam female connector are aligned with the liquid discharge male connector, the flushing male connector and the steam male connector respectively;
moving the liquid discharge male connector and the flushing male connector toward the liquid discharge female connector and the flushing female connector, such that the liquid discharge female connector and the flushing female connector are docked with the liquid discharge male connector and the flushing male connector respectively;
moving the steam male connector toward the steam female connector, such that the steam female connector is docked with the steam male connector.

### Beneficial Effects

The beneficial effects of this invention are as follows: in the waste collection device, the waste liquid collection and treatment system and the docking method for the waste liquid collection and treatment system of this invention, the waste liquid collection and treatment system can be cleaned by steam, avoiding the use of a large amount of hot water, and thus can be widely applied in various medical institutions with different conditions. In addition, the steam female connector and the steam male connector start to dock only after the liquid discharge female connector is docked with the liquid discharge male connector and the flushing female connector is docked with the flushing male connector, so that the liquid discharge connectors and the flushing connectors can position the docking of the steam connectors, which not only avoids the problem of low fault tolerance caused by simultaneous docking of the three sets of connectors, but also improves the docking success rate.

### BRIEF DESCRIPTION OF THE DRAWINGS

To more clearly illustrate the technical solutions of the embodiments of this invention, a brief introduction to the accompanying drawings required in the embodiments is provided below. It should be understood that the following drawings only illustrate certain embodiments of this invention and therefore shall not be regarded as limiting the scope. For those of ordinary skill in the art, other relevant drawings can be obtained based on these drawings without creative efforts.
FIG. 1 is a schematic structural view of a waste collection and treatment system according to an embodiment of this invention.
FIG. 2 is a partial structural schematic view of the waste collection device of the waste collection and treatment system shown in FIG. 1.
FIG. 3 is a top view of the trolley docking portion of the waste collection device shown in FIG. 2.
FIG. 4 is a cross-sectional view taken along line A-A in FIG. 3.
FIG. 5 is a cross-sectional view taken along line B-B in FIG. 3.
FIG. 6 is a schematic structural view of the docking head of the docking device of the waste collection and treatment system shown in FIG. 1.
FIG. 7 is an exploded schematic view of the docking head of the docking device shown in FIG. 6.
FIG. 8 is an assembly schematic view of partial components of the docking head of the docking device shown in FIG. 6.
FIG. 9 is an assembly schematic view of partial components of the docking head of the docking device shown in FIG. 6.
FIG. 10 is a front view of FIG. 9.
FIG. 11 is a cross-sectional view of FIG. 10.
FIG. 12 is a schematic structural view of the waste collection device and the docking device of the waste collection and treatment system shown in FIG. 1 before approaching each other.
FIG. 13 is a schematic view from another angle of FIG. 12.
FIG. 14 is a perspective schematic view of the waste collection device and the docking device of the waste collection and treatment system shown in FIG. 1 after approaching each other.
FIG. 15 is a front view of the waste collection device and the docking device of the waste collection and treatment system shown in FIG. 1 at the start of docking.
FIG. 16 is a cross-sectional view of FIG. 15.
FIG. 17 is a front view of the waste collection device and the docking device of the waste collection and treatment system shown in FIG. 1 after docking is completed.
FIG. 18 is a cross-sectional view of FIG. 17.

### DETAILED DESCRIPTION OF THE EMBODIMENTS

To further illustrate the technical means and effects adopted by this invention to achieve the intended purpose of the invention, the specific implementation modes, structures, features and effects of the display panel and waste collection device proposed according to this invention are described in detail below with reference to the accompanying drawings and preferred embodiments.

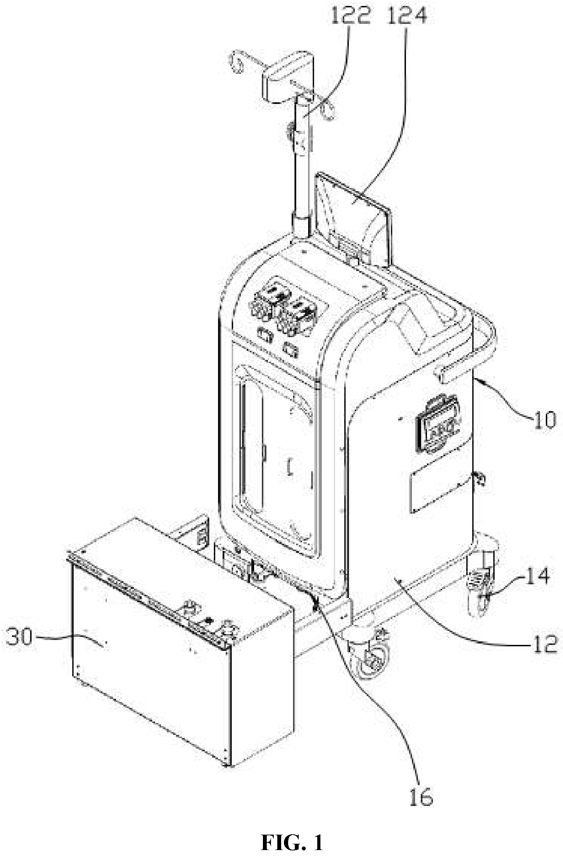

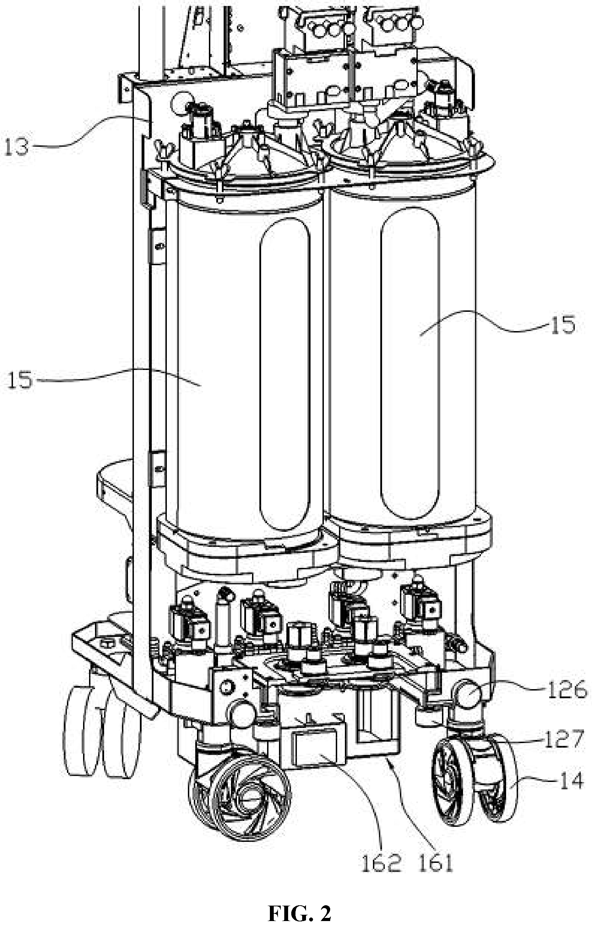

This invention provides a waste collection and treatment system, as shown in FIG. 1, which illustrates a waste collection and treatment system according to an embodiment. The waste collection and treatment system of this embodiment includes a waste collection device 10 and a docking device 30, and the docking device 30 is used for docking with the waste collection device 10. The waste collection device 10 can be connected to an external suction pipeline (not shown) via a consumable box 50, and the external suction pipeline can be connected to a suction connector (not shown). The suction connector can be a separate connector or attached to surgical equipment. The suction force of the suction pipeline can come from a vacuum source arranged inside the waste collection device 10 or from an external vacuum source. The suction force at the suction connector conveys waste into the waste collection device 10 for storage through the external suction pipeline. After docking with the waste collection device 10, the docking device 30 can empty the waste inside the waste collection device 10 through the docking device 30, and can form a flushing passage for cleaning the waste collection container 15 (see FIG. 2) of the waste collection device 10. The docking device 30 can also be connected to a steam generating device to perform steam cleaning on the waste collection device 10. The discharge circuit control, flushing circuit and steam cleaning circuit control of the waste collection and treatment system 10 are known to those skilled in the art and will not be repeated herein.

In this embodiment, the waste collection device 10 includes a trolley housing 12, a waste collection container (not shown) arranged inside the trolley housing 12, a trolley main frame 13, a traveling mechanism 14 arranged at the bottom of the trolley main frame 13, a waste collection container 15 arranged inside the trolley housing 12, and a trolley docking portion 16 mounted on the trolley main frame 13. The trolley housing 12 is further provided with a support hanging rod 122 and a collection-end display device 124.

Referring also to FIG. 2, in this embodiment, the trolley housing 12 of the waste collection device 10 is further provided with a magnetic stop block 126, and the magnetic stop block 126 is arranged on at least one side of the trolley docking portion 16.

Specifically, the trolley housing 12 is further provided with first anti-collision portions 127, and the first anti-collision portions 127 are located on both sides of the trolley docking portion 16. Specifically, the first anti-collision portions 127 can be anti-collision rollers.

Specifically, the waste collection container is used for accommodating waste sucked by the suction connector.

Specifically, the traveling mechanism 14 can include a plurality of rollers to facilitate the movement of the waste collection device 10, so that the waste collection device 10 forms a portable device for convenient use.

In this embodiment, the trolley docking portion 16 can be arranged at the bottom of the trolley main frame 13 for mating with the docking device 30. Specifically, the trolley docking portion 16 is provided with a mating space 161 for accommodating the mating head 34 of the docking device 30. The first anti-collision portions 127 are located on both sides of the mating space 161 to prevent the mating head 34 of the docking device 30 from colliding with the inner side walls of the mating space 161.

Specifically, a charging receiving portion 162 is further arranged inside the mating space 161. The charging receiving portion 162 can be a wireless charging receiving portion 162 to realize wireless charging. Of course, the charging receiving portion 162 can also be a wired plug-in part, as long as charging can be realized simultaneously when docking with the docking device 30.

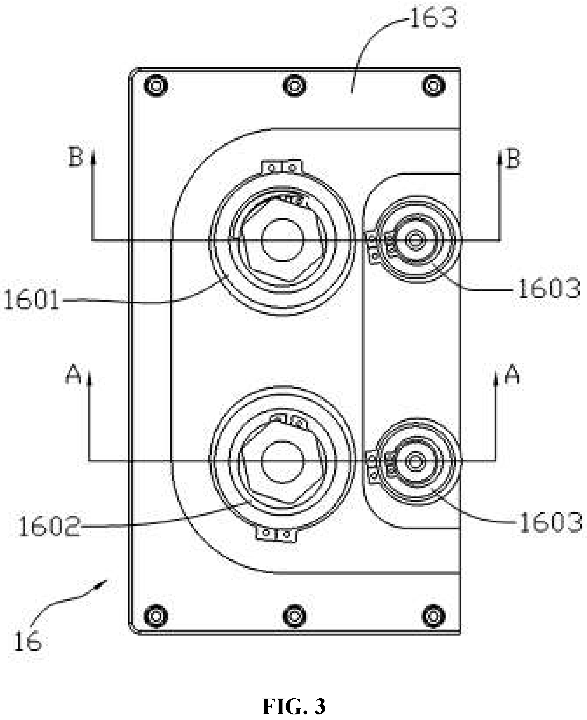

Referring to FIG. 3, in this embodiment, the trolley docking portion 16 includes a trolley connector mounting plate 163 and a plurality of collection connectors arranged on the trolley connector mounting plate 163. The plurality of collection connectors include a liquid discharge female connector 1601, a flushing female connector 1602, and a steam female connector 1603. The liquid discharge female connector 1601 and the flushing female connector 1602 are arranged to be floating relative to the trolley connector mounting plate 163. The liquid discharge female connector 1601 is used for discharging waste in the waste collection container 15, the flushing female connector 1602 is used for introducing cleaning liquid for cleaning the waste collection container 15, and the steam female connector 1603 is used for introducing steam to perform high-temperature cleaning on the waste collection container 15. The liquid discharge female connector 1601, the flushing female connector 1602 and the steam female connector 1603 are respectively used for one-to-one connection with a plurality of collection male connectors on the docking device 30. In this embodiment, the steam female connector 1603 can also be arranged to be floating relative to the trolley connector mounting plate 163; of course, the steam female connector 1603 can also be non-floating.

Specifically, there is one liquid discharge female connector 1601 and one flushing female connector 1602 respectively, and the liquid discharge female connector 1601 and the flushing female connector 1602 are arranged side by side in a direction perpendicular to the relative movement direction when the docking device 30 is docked with the waste collection device 10. There are two steam female connectors 1603, which are arranged side by side in a direction perpendicular to the relative movement direction when the docking device 30 is docked with the waste collection device 10, and the two steam female connectors 1603 are each connected to a waste collection container 15. The steam female connector 1603 is closer to the outer side of the waste collection device 10 than the liquid discharge female connector 1601 and the flushing female connector 1602, that is, when the waste collection device 10 is docked with the docking device 30, the docking device 30 approaches the steam female connector 1603 first. It can be understood that when there is one waste collection container 15, there is also one steam female connector 1603. Specifically, the steam female connector 1603 can be connected to the tank cover or tank bottom of the waste collection device 10 through a steam pipeline. The tank cover or tank bottom can be made of high-temperature resistant materials, and the steam pipeline can also be made of high-temperature resistant materials.

Specifically, the ends of the liquid discharge female connector 1601 and the flushing female connector 1602 for docking with the docking device 30 are flush, and the end of the steam female connector 1603 for docking with the docking device 30 is higher than the ends of the liquid discharge female connector 1601 and the flushing female connector 1602 for docking with the docking device 30. That is, before docking, the distance between the steam female connector 1603 and the docking device 30 is greater than the distance between the liquid discharge female connector 1601, the flushing female connector 1602 and the docking device 30.

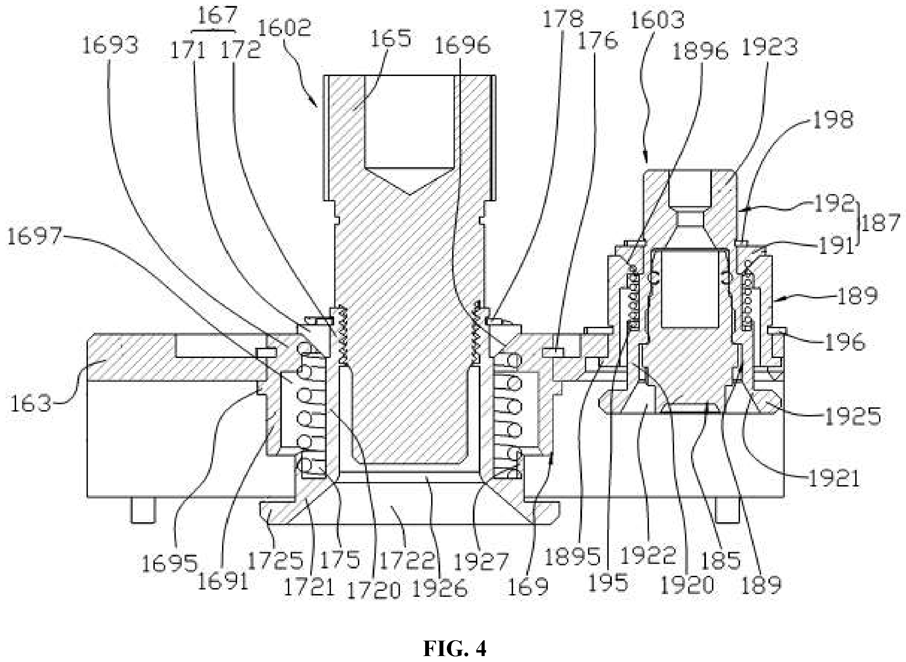

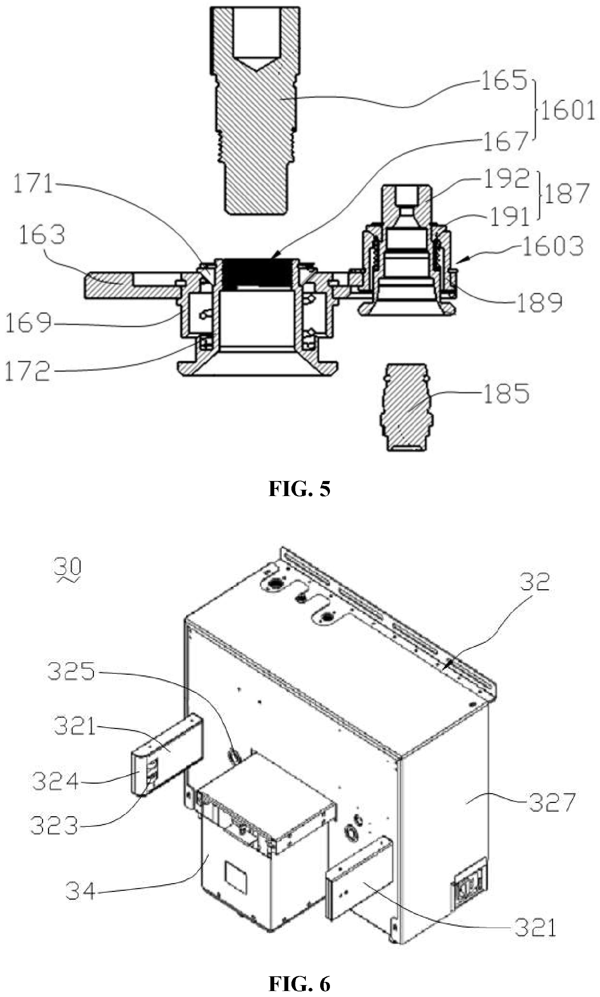

Referring also to FIGS. 4 and 5, in this embodiment, each of the liquid discharge female connector 1601 and the flushing female connector 1602 includes a collection-end connector 165 and a first floating connector 167. The collection-end connector 165 is connected to the waste collection container 15. The first floating connector 167 is sleeved on the collection-end connector 165 and mounted on the trolley connector mounting plate 163. The first floating connector 167 is used for guiding a docking-end connector of the docking device 30 to connect with the collection-end connector 165. When the first floating connector 167 is not correspondingly connected to the docking-end connector, the first floating connector 167 is at an initial position; during the connection of the docking-end connector, the first floating connector 167 can float and move relative to the trolley connector mounting plate 163 and guide the docking-end connector to align and engage with the collection-end connector 165 correspondingly. The interior of the collection-end connector 165 is hollow, so that both ends of the collection-end connector 165 are respectively communicated with the waste collection container 15 and the docking device 30.

Specifically, the trolley connector mounting plate 163 can be fixedly connected to the trolley main frame 13 through fasteners 168.

Specifically, the trolley docking portion 16 further includes a first floating connector mounting member 169, and the first floating connector 167 includes a first floating port 171 and a first floating guide member 172. The first floating connector mounting member 169 is fixed to the trolley connector mounting plate 163, and the first floating connector mounting member 169 includes a positioning portion 1691 extending into the mating space 161. The first floating guide member 172 is sleeved on the collection-end connector 165 and penetrates the first floating connector mounting member 169 to guide the docking-end connector of the docking device 30; the first floating port 171 is fixedly connected to the first floating guide member 172, and the first floating port 171 is floatingly matched with a mounting hole of the first floating connector mounting member 169 to engage with the first floating connector mounting member 169 in an initial state. The first floating connector 167 is floatingly arranged in the mounting hole formed by the first floating connector mounting member 169, so that the first floating connector 167 can swing in any direction within a certain range relative to the trolley connector mounting plate 163, thereby realizing guiding and position correction for the docking-end connector. When the docking-end connector of the docking device 30 is not connected, the first floating port 171 engages with the first floating connector mounting member 169, and the first floating connector 167 is at the initial position; during the connection of the docking-end connector, the first floating port 171 moves relative to the first floating connector mounting member 169 and guides the docking-end connector to align with the corresponding collection-end connector 165 and complete engagement to form a cleaning passage or a waste discharge passage.

Specifically, a first elastic member 175 is arranged between the first floating connector mounting member 169 and the first floating connector 167 to reset the first floating connector 167, so that the first floating port 171 is reset from a movable state to the state of engaging with the first floating connector mounting member 169. Specifically, two ends of the first elastic member 175 abut against the first floating connector mounting member 169 and the first floating guide member 172 respectively. The first elastic member 175 can be a compression spring. When the docking-end connector of the docking device 30 is not connected and the first floating connector 167 is at the initial position, the first elastic member 175 is in an initial state, that is, the first elastic member 175 is not compressed or only slightly compressed; during the connection of the docking-end connector, the first floating connector 167 floats and the first elastic member 175 is compressed; after the docking-end connector is removed, the elastic force accumulated by the compression of the first elastic member 175 restores the first floating connector 167 to the initial state.

Specifically, two mounting holes can be formed on the trolley connector mounting plate 163 for respectively mounting two first floating connector mounting members 169, so as to correspondingly arrange two first floating connectors 167, and two collection-end connectors 165 are respectively connected to the two first floating connectors 167.

Specifically, the collection-end connector 165 and the first floating connector 167 are fixedly connected to form a whole, and the outer walls of the collection-end connector 165 and the first floating connector 167 can be integrally formed or connected by threads.

Specifically, the first floating connector mounting member 169 includes a top mounting portion 1693, and the top mounting portion 1693 is fixedly connected to the trolley connector mounting plate 163. Specifically, a first limiting portion 1695 is formed at the junction of the top mounting portion 1693 and the positioning portion 1691, the first limiting portion 1695 abuts against one side surface of the trolley connector mounting plate 163, and a first clamping member 176 abutting against the trolley connector mounting plate 163 and clamping the top mounting portion 1693 of the first floating connector mounting member 169 is arranged on the other side surface of the trolley connector mounting plate 163. Thus, the top mounting portion 1693 is fixedly connected to the trolley connector mounting plate 163 through the first limiting portion 1695 and the first clamping member 176. Specifically, the first clamping member 176 can be a circlip. Specifically, a first matching hole 1696 is formed on the top mounting portion 1693, the first matching hole 1696 is a tapered hole with a large upper part and a small lower part, or the inner side wall of the first matching hole 1696 is an inclined plane, that is, at least part of the inner side wall of the first matching hole 1696 faces away from the mating space 161, and the size of the first matching hole 1696 gradually decreases. A cavity 1697 communicated with the first matching hole 1696 is formed inside the positioning portion 1691, and the first elastic member 175 is accommodated in the cavity 1697, with one end thereof abutting against the top mounting portion 1693. A first clamping groove for accommodating the first clamping member 176 is formed on the first floating connector mounting member 169.

Specifically, the first floating port 171 includes a matching surface for matching with the first matching hole 1696 of the first floating connector mounting member 169, and the matching surface is a tapered surface or an inclined plane, that is, the matching surface faces the mating space 161 and is at least partially inclined. The matching surface of the first floating port 171 can engage with the inner surface of the first matching hole 1696, or can disengage from the first matching hole 1696 when the first floating port 171 floats.

Specifically, the first floating guide member 172 includes a first sleeve portion 1720 and a first guide end 1721 for guiding the docking-end connector. One end of the first sleeve portion 1720 is connected to the collection-end connector 165, and the first guide end 1721 is connected to the other end of the first sleeve portion 1720. A first guide opening 1722 is formed in the first guide end 1721, the first guide opening 1722 can be a conical hole, or the inner side wall of the first guide opening 1722 is composed of a plurality of inclined planes, that is, the first guide opening 1722 is a bell-mouth structure, which can be a curved surface or a tapered surface. Thus, during docking, the first guide opening 1722 can guide the docking-end connector of the docking device 30, facilitating position alignment during docking. One end of the first floating guide member 172 extends from one end of the first floating connector mounting member 169 into the mating space 161, and the first floating guide member 172 further includes a first limiting edge 1725 cooperating with the positioning portion 1691 to limit an upward floating range of the first floating connector 167 during floating. The first limiting edge 1725 is arranged on the outer side wall of the first guide end 1721. In this embodiment, the outer diameter of the first limiting edge 1725 is larger than the inner diameter of the positioning portion 1691, and when the first floating guide member 172 moves upward to abut against the positioning portion 1691, the positioning portion 1691 can prevent the first floating guide member 172 from moving upward further. Specifically, a rounded corner 1726 is formed at the end of the first guide end 1721 away from the first limiting edge 1725. Specifically, the diameter of the smallest aperture position of the first guide opening 1722 can range from 40 mm to 60 mm, so that the size of the first guide opening 1722 is relatively large and the fault-tolerant space is large. In addition, the diameter of the first floating connector 167 ranges from 30 mm to 38 mm, and the inner diameter of the positioning portion 1691 ranges from 50 mm to 70 mm, so that the first floating connector 167 has a large inclination angle range before docking is completed, making it more convenient to guide the first floating connector 167 to dock.

Specifically, the first floating guide member 172 further includes an outer tube 1727, the outer tube 1727 is connected to the outer side wall of the first guide end 1721, and the outer tube 1727 is sleeved outside the first guide end 1721 with a gap formed therebetween. One end of the first elastic member 175 abuts at the gap between the outer tube 1727 and the first guide end 1721.

Specifically, a second clamping member 178 abutting against the first floating port 171 and clamping the first floating guide member 172 is arranged on one side surface of the first floating port 171. Thus, the first floating port 171 is fixedly connected to the first floating guide member 172 through the second clamping member 178. The second clamping member 178 can be a circlip. A second clamping groove for accommodating the second clamping member 178 is formed on the first floating guide member 172.

In this embodiment, the steam female connector 1603 includes a collection-end mounting base 185 and a second floating connector 187. The collection-end mounting base 185 is connected to the docking device 30, the second floating connector 187 is connected to the waste collection container 15, the collection-end mounting base 185 is sleeved inside the second floating connector 187, and the second floating connector 187 is mounted on the trolley connector mounting plate 163. The second floating connector 187 is also used for guiding the docking-end connector of the docking device 30 to connect with the collection-end mounting base 185. When the second floating connector 187 is not correspondingly connected to the docking-end connector, the second floating connector 187 is at an initial position; during the connection of the docking-end connector, the second floating connector 187 can float and move relative to the trolley connector mounting plate 163 and guide the docking-end connector to align and engage with the collection-end mounting base 185 correspondingly.

Specifically, the trolley docking portion 16 further includes a second floating connector mounting member 189, and the second floating connector 187 includes a second floating port 191 and a second floating guide member 192. The second floating connector mounting member 189 is fixed to the trolley connector mounting plate 163. The second floating guide member 192 is sleeved on the collection-end mounting base 185 and penetrates the second floating connector mounting member 189 to guide the docking-end connector of the docking device 30; the second floating port 191 is fixedly connected to the second floating guide member 192, and the second floating port 191 is floatingly matched with a mounting hole of the second floating connector mounting member 189 to engage with the second floating connector mounting member 189 in an initial state. The second floating connector 187 is floatingly arranged in the mounting hole formed by the second floating connector mounting member 189, so that the second floating connector 187 can swing in any direction within a certain range relative to the trolley connector mounting plate 163, thereby realizing guiding and position correction for the docking-end connector. When the docking-end connector of the docking device 30 is not connected, the second floating port 191 engages with the second floating connector mounting member 189, and the second floating connector 187 is at the initial position; during the connection of the docking-end connector, the second floating port 191 moves relative to the second floating connector mounting member 189 and guides the docking-end connector to align with the corresponding collection-end mounting base 185 and complete engagement to form a steam passage.

Specifically, a second elastic member 195 is arranged between the second floating connector mounting member 189 and the second floating connector 187 to reset the second floating connector 187, so that the second floating port 191 is reset from a movable state to the state of engaging with the second floating connector mounting member 189. Specifically, two ends of the second elastic member 195 abut against the second floating connector mounting member 189 and the second floating guide member 192 respectively. The second elastic member 195 can be a compression spring. When the docking-end connector of the docking device 30 is not connected and the second floating connector 187 is at the initial position, the second elastic member 195 is in an initial state, that is, the second elastic member 195 is not compressed or only slightly compressed; during the connection of the docking-end connector, the second floating connector 187 floats and the second elastic member 195 is compressed; after the docking-end connector is removed, the elastic force accumulated by the compression of the second elastic member 195 restores the second floating connector 187 to the initial state.

Specifically, two mounting holes can be formed on the trolley connector mounting plate 163 for respectively mounting two second floating connector mounting members 189, so as to correspondingly arrange two second floating connectors 187, and two collection-end mounting bases 185 are respectively connected to the two second floating connectors 187.

Specifically, the collection-end mounting base 185 and the second floating connector 187 are fixedly connected to form a whole, and the outer walls of the collection-end mounting base 185 and the second floating connector 187 can be integrally formed or connected by threads.

Specifically, the second floating connector mounting member 189 includes a bottom mounting portion 1893, and the bottom mounting portion 1893 is fixedly connected to the trolley connector mounting plate 163. Specifically, a second limiting portion 1895 is formed on the bottom mounting portion 1893, the second limiting portion 1895 abuts against one side surface of the trolley connector mounting plate 163, and a third clamping member 196 abutting against the trolley connector mounting plate 163 and clamping the bottom mounting portion 1893 of the second floating connector mounting member 189 is arranged on the other side surface of the trolley connector mounting plate 163. Thus, the bottom mounting portion 1893 is fixedly connected to the trolley connector mounting plate 163 through the second limiting portion 1895 and the third clamping member 196. Specifically, the third clamping member 196 can be a circlip. Specifically, a second matching hole 1896 is formed on the second floating connector mounting member 189, the second matching hole 1896 is a tapered hole with a large upper part and a small lower part, or the inner side wall of the second matching hole 1896 is an inclined plane, that is, at least part of the inner side wall of the second matching hole 1896 faces away from the mating space 161, and the size of the second matching hole 1896 gradually decreases. A third clamping groove for accommodating the third clamping member 196 is formed on the second floating connector mounting member 189.

Specifically, the second floating port 191 includes a matching surface for matching with the second matching hole 1896 of the second floating connector mounting member 189, and the matching surface is a tapered surface or an inclined plane, that is, the matching surface faces the mating space 161 and is at least partially inclined. The matching surface of the second floating port 191 can engage with the inner surface of the second matching hole 1896, or can disengage from the second matching hole 1896 when the second floating port 191 floats.

Specifically, the second floating guide member 192 includes a second sleeve portion 1920, a second guide end 1921 for guiding the docking-end connector, and a connecting portion 1923. The second guide end 1921 and the connecting portion 1923 are respectively connected to two ends of the second sleeve portion 1920, and the collection-end mounting base 185 is connected inside the second sleeve portion 1920. A second guide opening 1922 is formed in the second guide end 1921, the second guide opening 1922 can be a conical hole, or the inner side wall of the second guide opening 1922 is composed of a plurality of inclined planes, that is, the second guide opening 1922 is a bell-mouth structure, which can be a curved surface or a tapered surface. Thus, during docking, the second guide opening 1922 can guide the docking-end connector of the docking device 30, facilitating position alignment during docking. One end of the second floating guide member 192 extends from one end of the second floating connector mounting member 189 into the mating space 161, and the second floating guide member 192 further includes a second limiting edge 1925 cooperating with the bottom mounting portion 1893 to limit an upward floating range of the second floating connector 187 during floating. The second limiting edge 1925 is arranged on the outer side wall of the second guide end 1921. In this embodiment, the outer diameter of the second limiting edge 1925 is larger than the inner diameter of the bottom mounting portion 1893, and when the second floating guide member 192 moves upward to abut against the bottom mounting portion 1893, the bottom mounting portion 1893 can prevent the second floating guide member 192 from moving upward further.

Specifically, a fourth clamping member 198 abutting against the second floating port 191 and clamping the second floating guide member 192 is arranged on one side surface of the second floating port 191. Thus, the second floating port 191 is fixedly connected to the second floating guide member 192 through the fourth clamping member 198. The fourth clamping member 198 can be a circlip. A fourth clamping groove for accommodating the fourth clamping member 198 is formed on the second floating guide member 192.

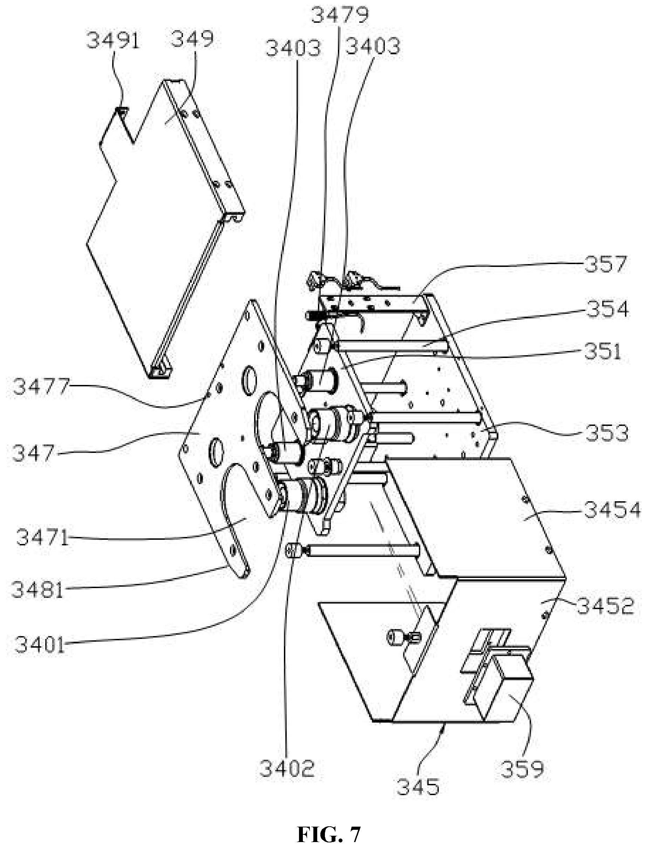

Referring to FIGS. 6 to 8, in this embodiment, the docking device 30 includes a liquid discharge station body 32 and a mating head 34, and the mating head 34 is arranged on one side of the liquid discharge station body 32. The mating head 34 is used for mating with the trolley docking portion 16 of the waste collection device 10. Specifically, the mating head 34 is inserted into the mating space 161 of the trolley docking portion 16 to realize preliminary alignment between the docking device 30 and the waste collection device 10.

In this embodiment, the liquid discharge station body 32 is further provided with liquid discharge station guide portions 321, and two liquid discharge station guide portions 321 are respectively arranged on both sides of the liquid discharge station body 32 to cooperate with both sides of the trolley housing 12 respectively, so as to guide the docking device 30 during the process when the mating head 34 of the docking device 30 enters the mating space 161. Specifically, second anti-collision portions 323 are further arranged on the mutually facing sides of the two liquid discharge station guide portions 321, and the second anti-collision portions 323 can be specifically anti-collision rollers to prevent damage caused by impact on the trolley housing 12 during docking. Specifically, a rounded corner portion 324 is formed at the end of the liquid discharge station guide portion 321 away from the liquid discharge station body 32 to better realize guiding.

Specifically, the liquid discharge station body 32 is further provided with a magnetic member 325. Specifically, the magnetic member 325 is arranged on one side of the mating head 34. When the docking device 30 contacts the waste collection device 10, the magnetic member 325 is attracted to the magnetic stop block 126 of the waste collection device 10, and at this time, the mating head 34 has completely entered the mating space 161.

Specifically, the liquid discharge station body 32 includes a liquid discharge station housing 327 and liquid discharge equipment arranged inside the liquid discharge station housing 327.

In this embodiment, the mating head 34 includes a plurality of docking connectors, and the plurality of docking connectors include a liquid discharge male connector 3401, a flushing male connector 3402 and a steam male connector 3403 respectively arranged corresponding to the liquid discharge female connector 1601, the flushing female connector 1602 and the steam female connector 1603 for respective docking. Specifically, the steam male connector 3403 can be connected to a steam generating device through a steam pipeline. The steam pipeline can be made of high-temperature resistant materials.

In this embodiment, the mating head 34 further includes an actuating assembly 343. The liquid discharge male connector 3401, the flushing male connector 3402 and the steam male connector 3403 are connected to the actuating assembly 343, and the actuating assembly 343 is arranged inside the mating head 34 and can move freely in a specific direction, thereby driving the liquid discharge male connector 3401, the flushing male connector 3402 and the steam male connector 3403 to move for docking with the collection-end connector 165 or the collection-end mounting base 185. After the mating head 34 is aligned with the trolley docking portion 16, the actuating assembly 343 operates to bring the liquid discharge male connector 3401 and the flushing male connector 3402 into contact with the first floating connector 167, and the steam male connector 3403 into contact with the second floating connector 187, and the docking-end connectors 34 cause the first floating connector 167 to float and move to guide the liquid discharge male connector 3401 and the flushing male connector 3402 to engage with the collection-end connector 165, and the second floating connector 187 to float and move to guide the steam male connector 3403 to engage with the collection-end mounting base 185. In this embodiment, the liquid discharge male connector 3401 is docked with one collection-end connector 165 to form a waste discharge passage for transporting waste in the waste collection container 15, the flushing male connector 3402 is docked with another collection-end connector 165 to form a passage for flushing the waste collection container 15 for transporting cleaning liquid for cleaning the waste collection container 15, and the steam male connector 3403 is docked with the collection-end mounting base 185 to form a passage for steam cleaning the waste collection container 15 for introducing steam for cleaning the waste collection container 15. Specifically, the liquid discharge male connector 3401 is connected to a cleaning interface on the liquid discharge station body 32 for filling cleaning liquid; the docking device 30 further includes a suction pump (not shown) for connecting with the liquid discharge male connector 3401, and the waste sucked by the liquid discharge male connector 3401 is discharged through a discharge connector on the liquid discharge station body 32. Specifically, the actuating assembly 343 can be a pneumatic pump, a stepper motor, or other components or mechanical structures capable of telescopic movement. In this embodiment, a pneumatic pump is preferably used, which can reduce circuit components of the docking device 30, thereby reducing circuit faults and potential risks to the equipment caused by complex circuits. In addition, the speed and force of the pneumatic pump during pushing are more conducive to the guiding effect of the first floating connector 167 on the liquid discharge male connector 3401 and the flushing male connector 3402, and the second floating connector 187 on the steam male connector 3403.

Specifically, rounded corners are provided at the ends of the liquid discharge male connector 3401 and the flushing male connector 3402 for docking with the collection-end connector 165 to guide during the docking process of the liquid discharge male connector 3401 and the flushing male connector 3402 with the first floating connector 167, thereby facilitating docking.

In this embodiment, the mating head 34 further includes a mating head housing 345, and the docking connectors and the actuating assembly 343 are arranged inside the mating head housing 345. The mating head housing 345 includes a front panel 3452 and side plates 3454 connected to both sides of the front panel 3452, and the rear end of the mating head housing 345 opposite to the front panel 3452 is connected to the liquid discharge station body 32.

In this embodiment, the mating head 34 further includes a mating plate 347, two guide slots 3471 are formed in the mating plate 347, and the liquid discharge female connector 1601 and the flushing female connector 1602 can correspondingly enter the guide slots 3471. Preferably, the guide slot 3471 is approximately U-shaped with an arc-shaped inner end and wedge-shaped side edges 3473, so that the opening width from the outer end to the inner end of the guide slot 3471 gradually decreases. Such a structure enables the first floating connector 167 to gradually align with the guide slot 3471 without collision during the process of pushing the mating head 34 into the mating space 161 of the trolley docking portion 16.

Specifically, an elastic member hanging hole 3477 is formed on the mating plate 347.

Specifically, the mating plate 347 is further provided with a first magnetic switch 3479 and a second magnetic switch 3480, both of which are arranged at the rear end of the mating plate 347 (i.e., the end away from the guide slot 3471).

In this embodiment, the mating head 34 further includes a cover plate 349, and the cover plate 349 is movably arranged above the mating plate 347. When the mating head 34 is pushed into the mating space 161, the waste collection device 10 pushes the cover plate 349 to stagger the cover plate 349 relative to the mating plate 347; after the mating head 34 is removed from the mating space 161, the cover plate 349 can reset to a position covering the mating plate 347 and all docking connectors.

Specifically, sliding grooves are formed on the cover plate 349, and guide rails 3481 slidably arranged in the sliding grooves are arranged on both sides of the mating plate 347 to guide the movement of the cover plate 349.

Specifically, a third elastic member (not shown) is arranged between the mating plate 347 and the cover plate 349, and the third elastic member is used for providing elastic force for automatically resetting the cover plate 349 after the mating head 34 is removed from the mating space 161. More specifically, during the process of pushing the mating head 34 into the mating space 161, the third elastic member is gradually stretched, and retracts to release elastic force after the mating head 34 is removed from the mating space 161. Specifically, an elastic member hanging portion 3491 is arranged on the cover plate 349, and two ends of the third elastic member are respectively connected to the elastic member hanging portion 3491 and the elastic member hanging hole 3477 on the mating plate 347.

Specifically, the cover plate 349 is further provided with a first magnetic element 3493 and a second magnetic element 3494. The first magnetic element 3493 is arranged at the rear end of the cover plate 349 (i.e., the end of the cover plate 349 facing away from the trolley docking portion 16), and the second magnetic element 3494 is arranged at the front end of the cover plate 349 (i.e., the end of the cover plate 349 facing the trolley docking portion 16). When the first magnetic switch 3479 senses the first magnetic element 3493, it indicates that the docking device 30 is ready to dock with the waste treatment device 10; when the second magnetic switch 3480 senses the second magnetic element 3494, it indicates that the mating head 34 has completely entered the mating space 161, and the first actuating assembly can start working for docking.

In this embodiment, the mating head 34 further includes a moving plate 351. The liquid discharge male connector 3401, the flushing male connector 3402 and the steam male connector 3403 are all arranged on the moving plate 351. The actuating assembly 343 is connected to the moving plate 351 and drives the moving plate 351 to move up and down, thereby driving the liquid discharge male connector 3401, the flushing male connector 3402 and the steam male connector 3403 to move for docking with the collection-end connector 165 or the collection-end mounting base 185.

In this embodiment, the mating head 34 further includes a mating bottom plate 353, support columns 354, and guide columns 355. The moving plate 351 is located between the mating bottom plate 353 and the mating plate 347. The support columns 354 and the guide columns 355 are both fixed relative to the mating bottom plate 353, the mating plate 347 is fixedly connected to the support columns 354, and the moving plate 351 is movably penetrated by the guide columns 355. Specifically, one end of the guide column 355 is also connected to the mating plate 347. Buffer pads 356 are arranged at the ends of the support columns 354 and the guide columns 355 connected to the mating plate 347 to reduce vibration and noise when the moving plate 351 rises to a position close to the mating plate 347. One end of the support column 354 located between the two guide slots 3471 is connected to the mating head housing 345, and one end of the remaining support columns 354 is connected to the mating bottom plate 353.

Specifically, bushings 3552 are sleeved on the guide columns 355 to reduce friction between the moving plate 351 and the guide columns 355 when the moving plate 351 moves up and down.

In this embodiment, the mating head 34 further includes a sensor bracket 357, and a first sensor 3571 and a second sensor 3573 arranged at intervals are arranged on the sensor bracket 357. A sensor block 3511 is arranged on the moving plate 351, and the first sensor 3571 and the second sensor 3573 are used for sensing with the sensor block 3511. When the first sensor 3571 senses the sensor block 3511, it indicates that the collection-end connectors 165 are successfully docked with the liquid discharge male connector 3401 and the flushing male connector 3402; when the work of the liquid discharge station body 32 is completed, the actuating assembly 343 drives the moving plate 351, the liquid discharge male connector 3401 and the flushing male connector 3402 to descend, and the sensor block 3511 moves away from the first sensor 3571 until the second sensor 3573 senses the sensor block 3511, indicating that the moving plate 351, the liquid discharge male connector 3401 and the flushing male connector 3402 return to the initial position. The first sensor 3571 and the second sensor 3573 can be photoelectric sensors. When the first sensor 3571 senses the sensor block 3511, the first sensor 3571 lights up; when the second sensor 3573 senses the sensor block 3511, the second sensor 3573 lights up. It can be understood that the sensor bracket 357 can also be omitted, and the first sensor 3571 and the second sensor 3573 can be arranged on other structures (such as the mating head housing 345), as long as the first sensor 3571 and the second sensor 3573 are fixed relative to the mating plate 347.

In this embodiment, the mating head 34 further includes a charging port 359 for charging the waste collection device 10 through the charging receiving portion 162 of the trolley docking portion 16. Specifically, the charging port 359 can be a wireless charging port, which can charge by contacting or approaching the charging receiving portion 162.

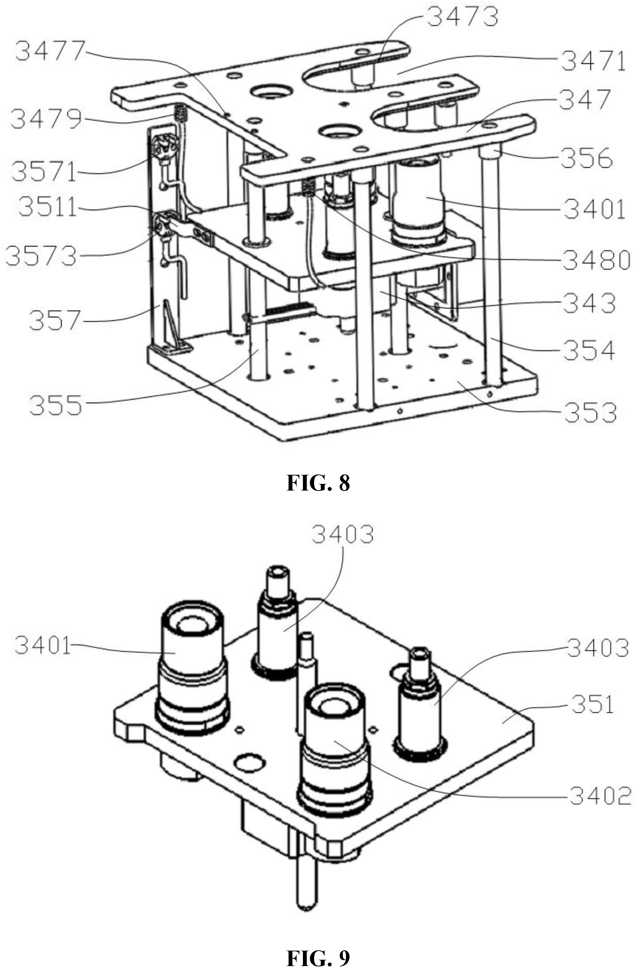

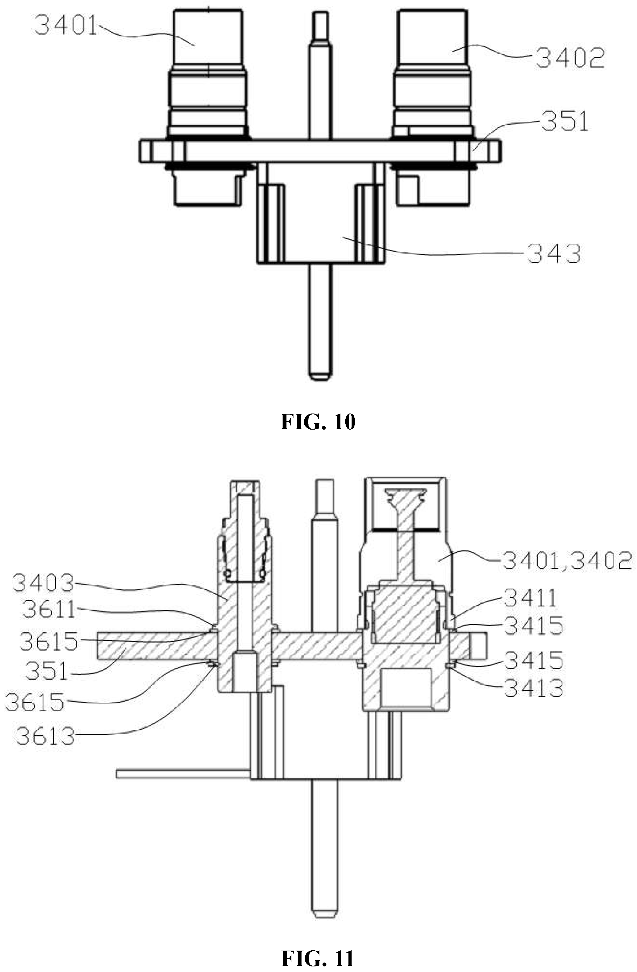

In this embodiment, referring to FIGS. 9 to 11, first step portions 3411 are arranged on the outer side walls of the liquid discharge male connector 3401 and the flushing male connector 3402. One end of each of the liquid discharge male connector 3401 and the flushing male connector 3402 passes through the moving plate 351, the first step portion 3411 abuts against one side surface of the moving plate 351, and a first fastener 3413 is clamped on the liquid discharge male connector 3401 or the flushing male connector 3402 and abuts against the other side surface of the moving plate 351, thereby fixing the liquid discharge male connector 3401 and the flushing male connector 3402 on the moving plate 351. Specifically, first gaskets 3415 are further arranged between the first step portion 3411 and the moving plate 351, and between the first fastener 3413 and the moving plate 351.

Specifically, a second step portion 3611 is arranged on the outer side wall of the steam male connector 3403. One end of the steam male connector 3403 passes through the moving plate 351, the second step portion 3611 abuts against one side surface of the moving plate 351, and a second fastener 3613 is clamped on the steam male connector 3403 and abuts against the other side surface of the moving plate 351, thereby fixing the steam male connector 3403 on the moving plate 351. Specifically, second gaskets 3615 are further arranged between the second step portion 3611 and the moving plate 351, and between the second fastener 3613 and the moving plate 351. Specifically, the first fastener 3413 and the second fastener 3613 can be circlips, and the first gasket 3415 and the second gasket 3615 can be PTFE gaskets.

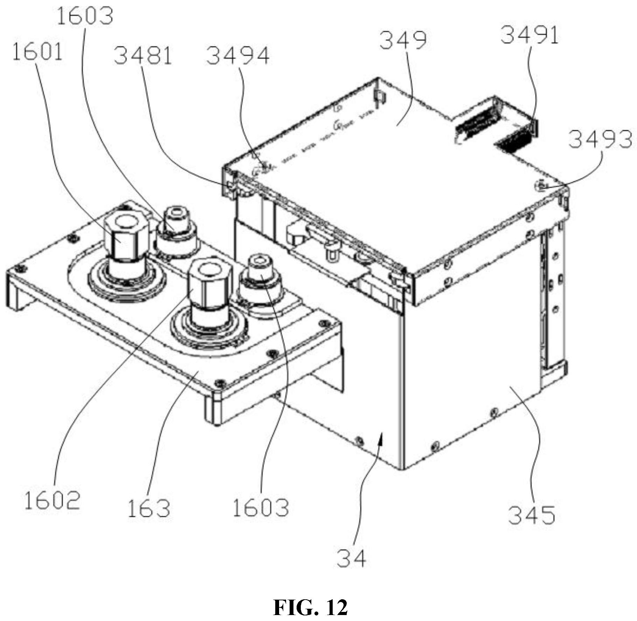

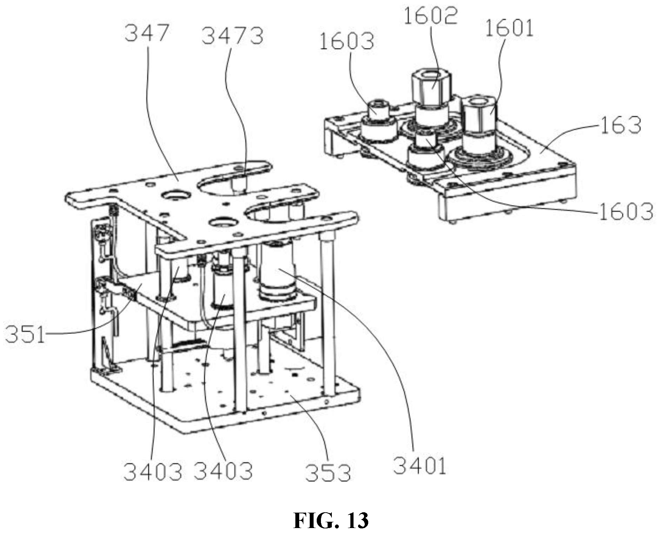

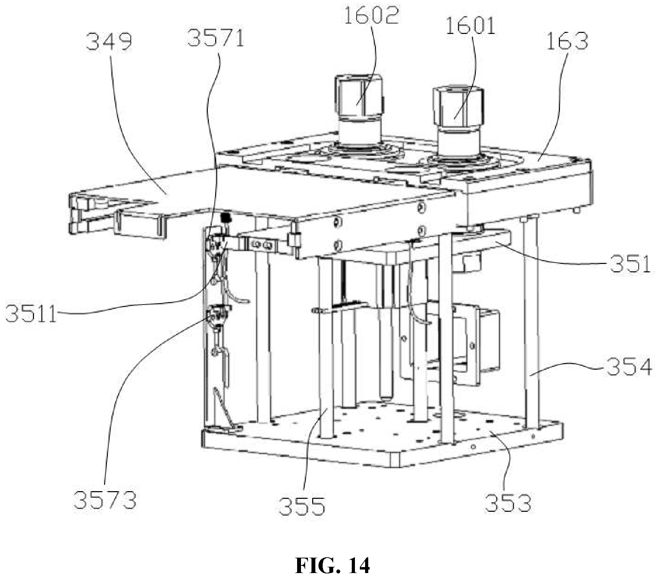

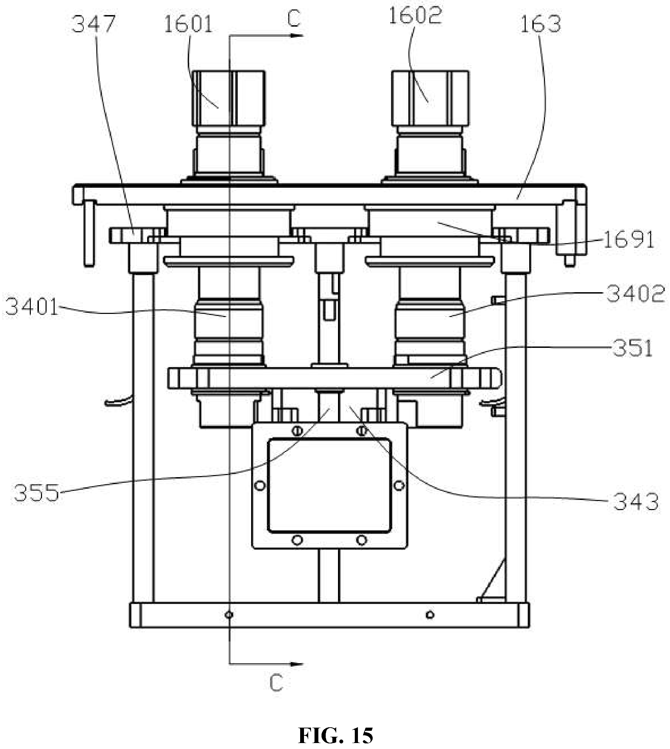

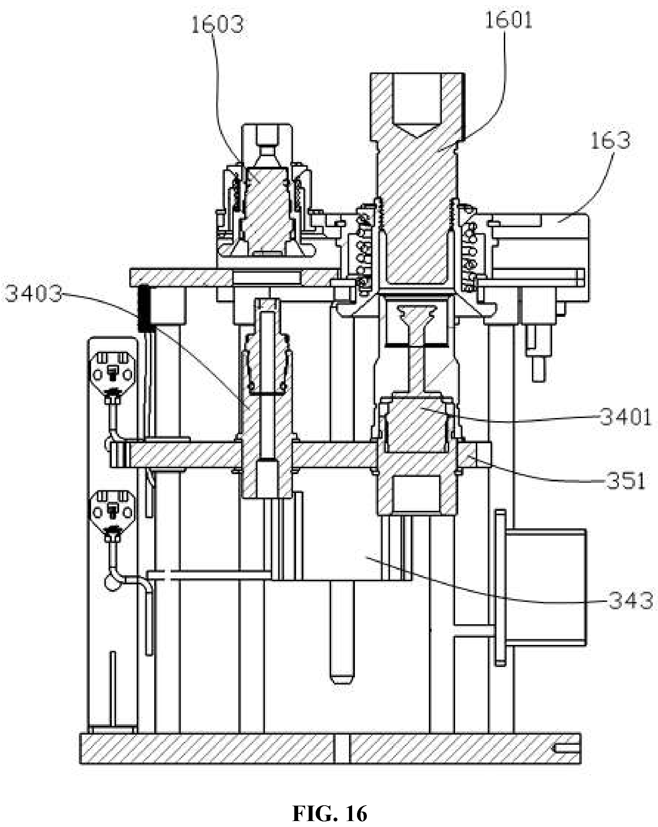

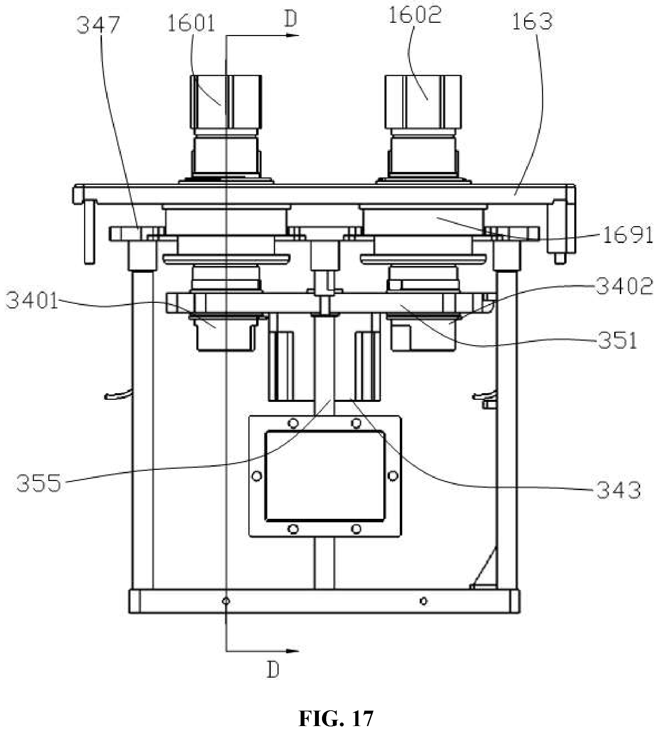

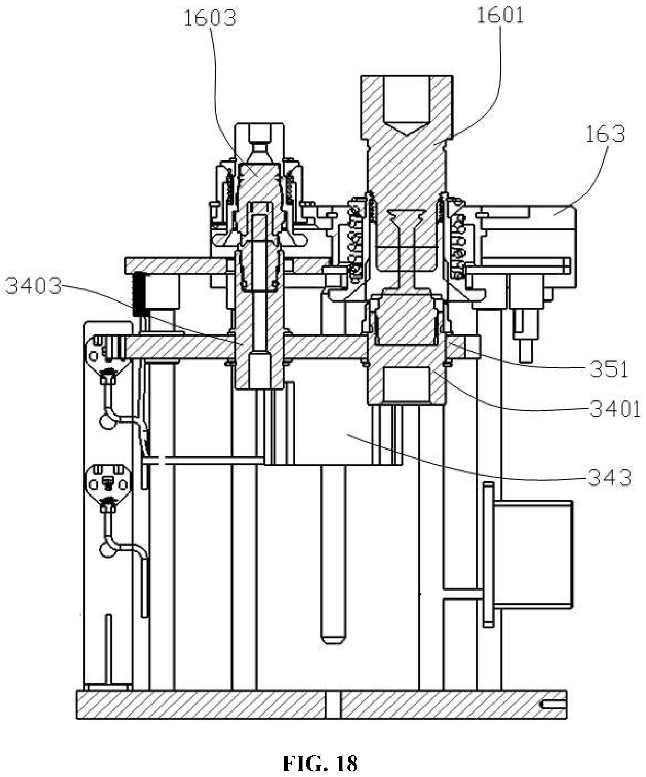

In this embodiment, referring to FIGS. 12 to 14, when the mating head 34 has not entered the mating space 161 of the trolley docking portion 16, the first floating connector mounting member 169 is located on one side of the mating head 34, and the cover plate 349 covers the mating plate 347. Then, the mating head 34 gradually extends into the mating space 161 of the trolley docking portion 16. After the trolley connector mounting plate 163 contacts the cover plate 349, as the mating head 34 moves into the mating space 161, the trolley connector mounting plate 163 gradually pushes the cover plate 349 to move relative to the mating plate 347. During this process, referring to FIGS. 15 and 16, the first floating connector 167 gradually enters the guide slot 3471 until the collection-end connector 165 is roughly aligned with the liquid discharge male connector 3401 or the flushing male connector 3402. The guide slot 3471 plays a guiding role in the entry of the first floating connector 167, and at this time, the positioning portion 1691 of the first floating connector mounting member 169 cooperates with the guide slot 3471 of the mating plate 347 to realize preliminary positioning. Next, the actuating assembly 343 is activated, and the actuating assembly 343 operates to bring the liquid discharge male connector 3401 and the flushing male connector 3402 into contact with the two first floating connectors 167 respectively, and make the first floating connectors 167 float to guide the liquid discharge male connector 3401 and the flushing male connector 3402 to engage with the two collection-end connectors 165 respectively. Finally, referring to FIGS. 17 and 18, the actuating assembly 343 continues to work, driving the moving plate 351 to move upward further, thereby driving the steam male connector 3403 to contact the second floating connector 187, and making the second floating connector 187 float to guide the steam male connector 3403 to engage with the collection-end mounting base 185.

In this embodiment, the waste liquid collection and treatment system further includes a steam generating device, and the steam generating device is communicated with the steam male connector 3403.

In this embodiment, the waste liquid collection and treatment system can be cleaned by steam, avoiding the use of a large amount of hot water, and can be widely applied in various medical institutions with different conditions. In addition, the steam female connector 1603 starts to dock with the steam male connector 3403 only after the liquid discharge female connector 1601 is docked with the liquid discharge male connector 3401 and the flushing female connector 1602 is docked with the flushing male connector 3402. Therefore, the liquid discharge connectors and the flushing connectors can play a positioning role for the docking of the steam connectors, which not only avoids the problem of low fault tolerance caused by simultaneous docking of the three sets of connectors, but also improves the docking success rate. Of course, when the precision of each component meets the requirements, the three sets of connectors can also be docked simultaneously.

In this embodiment, since the positioning portion 1691 is provided on the first floating connector mounting member 169, after the positioning portion 1691 is positioned in the guide slot 3471, the floating space of the first floating connector 167 has no limiting relationship with the mating plate 347 and the guide slot 3471, thereby not restricting the floating space of the first floating connector 167, resulting in a large fault-tolerant space during docking, and the first floating connector 167 will not get stuck during the docking process. Before docking is completed, the horizontal floating space of the first floating connector 167 is related to the distance between the inner surface of the first floating connector mounting member 169 and the outer surface of the first floating guide member 172, and the vertical floating space of the first floating connector 167 is related to the distance between the lowermost surface of the first floating connector mounting member 169 and the first limiting edge 1725 of the first floating guide member 172. The larger these two distances are, the larger the floating space is. The specific distances can be set according to the actual size of the equipment and the average conditions of common places. The floating space can be completely unrestricted by the mating head 34 of the liquid discharge station, and a very large floating range can be realized by increasing these two distances if necessary.

This invention further provides a docking method for a waste liquid collection and treatment system, which is used for docking the waste collection device 10 and the docking device 30 of the above waste liquid collection and treatment system. In this embodiment, the docking method for a waste liquid collection and treatment system includes:
S11: moving the waste collection device 10 and the docking device 30 toward each other;
S13: aligning the mating head 34 with the trolley docking portion 16, so that the liquid discharge female connector 1601, the flushing female connector 1602 and the steam female connector 1603 are aligned with the liquid discharge male connector 3401, the flushing male connector 3402 and the steam male connector 3403 respectively;
S15: moving the liquid discharge male connector 3401 and the flushing male connector 3402 toward the liquid discharge female connector 1601 and the flushing female connector 1602, so that the liquid discharge female connector 1601 and the flushing female connector 1602 are docked with the liquid discharge male connector 3401 and the flushing male connector 3402 respectively;
S17: moving the steam male connector 3403 toward the steam female connector 1603, so that the steam female connector 1603 is docked with the steam male connector 3403.

It can be understood that, without conflict, the structures or structural features involved in the above embodiments may be combined arbitrarily.

In this description, unless otherwise expressly specified and limited, the terms "mounted", "coupled" and "connected" shall be interpreted in a broad sense. For example, they may refer to a fixed connection, a detachable connection, or an integral connection; they may refer to a mechanical connection or an electrical connection; they may refer to a direct connection, or an indirect connection through an intermediate medium, or an internal connection between two elements. For those of ordinary skill in the art, the specific meanings of the above terms may be understood according to specific circumstances.

In this description, the orientation or positional relationships indicated by the terms "upper", "lower", "front", "rear", "left", "right", "top", "bottom", "inner", "outer", "vertical", "horizontal" and the like are based on the orientation or positional relationships shown in the accompanying drawings, which are only for the clarity of expression of the technical solution and convenience of description, and therefore shall not be construed as limiting this invention.

In this description, the terms "include", "comprise" or any other variation thereof are intended to cover a non-exclusive inclusion, such that a process, method, article or apparatus that includes a list of elements may include not only those elements expressly listed, but also other elements not expressly listed.

The above are merely specific embodiments of this invention, but the protection scope of this invention is not limited thereto. Any person skilled in the art can easily think of changes or substitutions within the technical scope disclosed in this invention, which shall fall within the protection scope of this invention. Therefore, the protection scope of this invention shall be subject to the protection scope defined by the claims.

## Claims

1. A waste collection device for docking with a docking device (30), wherein the waste collection device (10) comprises a trolley docking portion (16), the trolley docking portion (16) comprises a trolley connector mounting plate (163) and a plurality of collection connectors arranged on the trolley connector mounting plate (163), the plurality of collection connectors comprise a liquid discharge female connector (1601), a flushing female connector (1602) and a steam female connector (1603), and the liquid discharge female connector (1601) and the flushing female connector (1602) are arranged to be floating relative to the trolley connector mounting plate (163).

2. The waste collection device according to claim **1,** wherein the steam female connector (1603) is arranged to be floating relative to the trolley connector mounting plate (163).

3. The waste collection device according to claim **1,** wherein there is one liquid discharge female connector (1601) and one flushing female connector (1602) respectively, the liquid discharge female connector (1601) and the flushing female connector (1602) are arranged side by side in a direction perpendicular to the relative movement direction when the docking device (30) is docked with the waste collection device (10), and there are two steam female connectors (1603) arranged side by side in a direction perpendicular to the relative movement direction when the docking device (30) is docked with the waste collection device (10).

4. The waste collection device according to claim **1,** wherein the steam female connector (1603) is closer to an outer side of the waste collection device (10) than the liquid discharge female connector (1601) and the flushing female connector (1602).

5. The waste collection device according to claim **1,** wherein the ends of the liquid discharge female connector (1601) and the flushing female connector (1602) for docking with the docking device (30) are flush, and the end of the steam female connector (1603) for docking with the docking device (30) is higher than the ends of the liquid discharge female connector (1601) and the flushing female connector (1602) for docking with the docking device (30).

6. The waste collection device according to claim **2**, wherein each of the liquid discharge female connector (1601) and the flushing female connector (1602) comprises a collection-end connector (165) and a first floating connector (167), the first floating connector (167) is sleeved on the collection-end connector (165) and mounted on the trolley connector mounting plate (163), and the first floating connector (167) is movable in a floating manner relative to the trolley connector mounting plate (163); the steam female connector (1603) comprises a collection-end mounting base (185) and a second floating connector (187), the collection-end mounting base (185) is sleeved inside the second floating connector (187), the second floating connector (187) is mounted on the trolley connector mounting plate (163), and the second floating connector (187) is movable in a floating manner relative to the trolley connector mounting plate (163).

7. The waste collection device according to claim **6**, wherein the trolley docking portion (16) is provided with a mating space (161) for accommodating a mating head (34) of the docking device (30); the collection-end connector (165) and the first floating connector (167) are fixedly connected to form a whole, the trolley docking portion (16) further comprises a first floating connector mounting member (169), the first floating connector (167) comprises a first floating port (171) and a first floating guide member (172), the first floating port (171) is fixedly connected to the first floating guide member (172), the first floating connector mounting member (169) is fixed to the trolley connector mounting plate (163), the first floating guide member (172) is sleeved on the collection-end connector (165) and penetrates the first floating connector mounting member (169), the first floating port (171) is floatingly matched with a mounting hole of the first floating connector mounting member (169), and a first elastic member (175) is arranged between the first floating connector mounting member (169) and the first floating connector (167); the trolley docking portion (16) further comprises a second floating connector mounting member (189), the second floating connector (187) comprises a second floating port (191) and a second floating guide member (192), the collection-end mounting base (185) and the second floating connector (187) are fixedly connected to form a whole, the second floating connector mounting member (189) is fixed to the trolley connector mounting plate (163), the second floating guide member (192) is sleeved on the collection-end mounting base (185) and penetrates the second floating connector mounting member (189), the second floating port (191) is fixedly connected to the second floating guide member (192), and a second elastic member (195) is arranged between the second floating connector mounting member (189) and the second floating connector (187).

8. The waste collection device according to claim **7**, wherein a first matching hole (1696) is formed on the first floating connector mounting member (169), the first floating port (171) comprises a matching surface for matching with the first matching hole (1696) of the first floating connector mounting member (169), and the first matching hole (1696) is a tapered hole with a large upper part and a small lower part, or an inner side wall of the first matching hole (1696) is an inclined plane; a second matching hole (1896) is formed on the second floating connector mounting member (189), the second floating port (191) comprises a matching surface for matching with the second matching hole (1896) of the second floating connector mounting member (189), and the second matching hole (1896) is a tapered hole with a large upper part and a small lower part, or an inner side wall of the second matching hole (1896) is an inclined plane.

9. The waste collection device according to claim **7**, wherein the first floating guide member (172) comprises a first sleeve portion (1720) and a first guide end (1721), one end of the first sleeve portion (1720) is connected to the collection-end connector (165), the first guide end (1721) is connected to the other end of the first sleeve portion (1720), a first guide opening (1722) is formed in the first guide end (1721), and the first guide opening (1722) is a bell-mouth structure; the second floating guide member (192) comprises a second sleeve portion (1920), a second guide end (1921) and a connecting portion (1923), the second guide end (1921) and the connecting portion (1923) are respectively connected to two ends of the second sleeve portion (1920), the collection-end mounting base (185) is connected inside the second sleeve portion (1920), a second guide opening (1922) is formed in the second guide end (1921), and the second guide opening (1922) is a bell-mouth structure.

10. The waste collection device according to claim **9**, wherein the first floating connector mounting member (169) comprises a positioning portion (1691) extending into the mating space (161), the first floating guide member (172) further comprises a first limiting edge (1725) cooperating with the positioning portion (1691) to limit an upward floating range of the first floating connector (167) during floating, and the first limiting edge (1725) is arranged on an outer side wall of the first guide end (1721); or, the first floating guide member (172) further comprises an outer tube (1727), the outer tube (1727) is connected to the outer side wall of the first guide end (1721), and the outer tube (1727) is sleeved outside the first guide end (1721) with a gap formed therebetween.

11. A waste liquid collection and treatment system, comprising a waste collection device (10) and a docking device (30) for docking with the waste collection device, wherein the waste collection device (10) is the waste collection device according to any one of claims **1** to **10**, the docking device (30) comprises a mating head (34), the mating head (34) comprises a plurality of docking connectors, and the plurality of docking connectors comprise a liquid discharge male connector (3401), a flushing male connector (3402) and a steam male connector (3403) arranged corresponding to the liquid discharge female connector (1601), the flushing female connector (1602) and the steam female connector (1603) respectively.

12. The waste liquid collection and treatment system according to claim **11**, wherein the mating head (34) further comprises a mating plate (347), two guide slots (3471) for the liquid discharge female connector (1601) and the flushing female connector (1602) to enter are formed in the mating plate (347), the guide slot (3471) is U-shaped, an inner end of the guide slot (3471) is arc-shaped, and side edges (3473) of the guide slot (3471) are wedge-shaped.

13. The waste liquid collection and treatment system according to claim **11**, wherein the mating head (34) further comprises a moving plate (351) and an actuating assembly (343), the liquid discharge male connector (3401), the flushing male connector (3402) and the steam male connector (3403) are all arranged on the moving plate (351), and the actuating assembly (343) is connected to the moving plate (351) and configured to drive the moving plate (351) to move up and down.

14. A docking method for a waste liquid collection and treatment system, for docking the waste collection device (10) and the docking device (30) of the waste liquid collection and treatment system according to any one of claims **11** to **13**, wherein the docking method for a waste liquid collection and treatment system comprises:
moving the waste collection device (10) and the docking device (30) toward each other;
aligning the mating head (34) with the trolley docking portion (16), such that the liquid discharge female connector (1601), the flushing female connector (1602) and the steam female connector (1603) are aligned with the liquid discharge male connector (3401), the flushing male connector (3402) and the steam male connector (3403) respectively;
moving the liquid discharge male connector (3401) and the flushing male connector (3402) toward the liquid discharge female connector (1601) and the flushing female connector (1602), such that the liquid discharge female connector (1601) and the flushing female connector (1602) are docked with the liquid discharge male connector (3401) and the flushing male connector (3402) respectively;
moving the steam male connector (3403) toward the steam female connector (1603), such that the steam female connector (1603) is docked with the steam male connector (3403).
